(19) Europäisches Patentamt
European Patent Office
Office européen des brevets

(11) **EP 3 742 702 B1**

(12) **EUROPEAN PATENT SPECIFICATION**

(45) Date of publication and mention
of the grant of the patent:
**21.02.2024 Bulletin 2024/08**

(21) Application number: **18900998.8**

(22) Date of filing: **08.08.2018**

(51) International Patent Classification (IPC):
*A61N 5/10* (2006.01)        *G16H 20/40* (2018.01)
*G16H 30/20* (2018.01)       *G16H 70/20* (2018.01)

(52) Cooperative Patent Classification (CPC):
**A61N 5/1031; A61N 5/00; G06Q 10/04;
G16H 20/40; G16H 30/20; G16H 70/20; H04L 65/40**

(86) International application number:
**PCT/CN2018/099445**

(87) International publication number:
**WO 2019/140886 (25.07.2019 Gazette 2019/30)**

(54) **STANDARDIZED CLOUD RADIOTHERAPY PLANNING METHOD AND STORAGE MEDIUM**

VERFAHREN ZUR PLANUNG EINER STANDARDISIERTEN CLOUD-STRAHLENTHERAPIE UND
SPEICHERMEDIUM

PROCÉDÉ DE PLANIFICATION DE RADIOTHÉRAPIE EN NUAGE STANDARDISÉE ET SUPPORT
DE STOCKAGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **19.01.2018  CN 201810054052**

(43) Date of publication of application:
**25.11.2020 Bulletin 2020/48**

(73) Proprietor: **Beijing Linking Medical Technology
Co., Ltd
Beijing 100085 (CN)**

(72) Inventor: **LI, Gui
Shanghai 201821 (CN)**

(74) Representative: **Dr. Gassner & Partner mbB
Wetterkreuz 3
91058 Erlangen (DE)**

(56) References cited:
**WO-A1-2017/133654       WO-A1-2017/133654
WO-A1-2017/174189       CN-A- 105 688 335
CN-A- 105 893 772        CN-A- 107 480 416
US-A1- 2017 143 994**

## Description

TECHNICAL FIELD

The invention pertains to the technical field of radiotherapy and cloud computing, relating to a standardized cloud radiotherapy planning method and storage medium.

## BACKGROUND OF THE INVENTION

On February 3, 2014, the "World Cancer Report 2014" published by the World Health Organization (WHO) pointed out that there are currently about 14 million new cancer patients each year, and it is expected to rise to 22 million in the next 20 years; within the same period, the death toll will rise from 8.2 million to 13 million each year. In its fact sheet 2017, WHO noted that globally, nearly one in six deaths was caused by cancer. Among them, 60% to 70% of tumor patients currently need to receive different types of radiation therapy (referred to as radiotherapy) at a certain stage of their entire disease treatment process.

According to statistics, the number of cancer incidence and death in China has ranked first in the world. In 2015, there were 4.3 million new cases of cancer and 2.8 million deaths in China. Radiotherapy together with surgical treatment and chemical treatment are the three major methods of tumor treatment. The current shortage of physicists in China is 10,000, and medical resources are extremely scarce.

On the one hand, the brand effect of the top three hospitals has attracted a large number of patients to flock to the top three hospitals for treatment, especially the large top three hospitals are overcrowded, and the bed utilization rate has been saturated for a long time. According to statistics, the bed utilization rate of primary-level medical institutions is only about 60%, and some medical resources are wasted, and the expected benefits have not been exerted. A large number of patients are concentrated in tertiary hospitals for diagnosis and treatment, which will inevitably cause overburden of medical staff and tight radiotherapy equipment and treatment beds, which cannot meet the needs of all patients. The excessive concentration of high-quality medical resources in tertiary hospitals has also led to a decline in medical service coverage and remote It is difficult for people in the area to get medical treatment from experienced experts nearby. If a patient has a treatment plan determined by an experienced doctor or physicist in a superior hospital, and then returns to the primary hospital for radiation treatment, not only the quality of diagnosis and treatment is guaranteed, but also there is no need to wait in line or wait long for beds and radiotherapy equipment.

On the other hand, the types of radiotherapy equipment in some hospitals are different, and may include several brands of radiotherapy equipment of multiple models. If a radiotherapy plan is prepared based on a radiotherapy equipment in advance and the equipment fails unexpectedly without the same model When replacing equipment, it will inevitably cause the shelving of previously prepared radiotherapy plans to delay the treatment time of the patient; or the radiotherapy equipment of the currently established radiotherapy plan has been occupied and needs to be queued for a long time, while other radiotherapy equipment is idle or waiting in line Shorter. Therefore, how to avoid the delay or delay of the radiotherapy plan caused by the failure of the radiotherapy equipment, improve the effective utilization of the radiotherapy equipment in the tertiary hospitals, reduce the waiting time of patients, divert the patients in the tertiary hospitals to the primary medical institutions and maintain the radiotherapy Level is a problem that needs to be solved urgently in the prior art. Document WO 2017/133654 discloses a cloud-based radiotherapy planning method wherein a standard radiotherapy plan,is converted into a specific radiotherapy plan that matches specific radiotherapy equipment.

## SUMMARY OF THE INVENTION

The purpose of the present invention is to provide a standardized cloud radiotherapy planning method, storage medium and system in order to overcome the above shortcomings of the prior art.

The invention is defined in the following claims. Other embodiments, equivalents, examples etc. are not a part of the invention.

A standardized cloud radiotherapy planning method suitable for execution in a standardized cloud radiotherapy planning system,comprises the following steps:(1) patient data is uploaded to a master cloud server, wherein the patient data comprises a patient image and medical order data;(2) a target area is delineated on the basis of the patient image;(3) the master cloud server decomposes a computation task and assigns it to a controlled computer, and the controlled computer uses a standard radiotherapy equipment mode to compute a radiotherapy plan for a patient to generate a standard radiotherapy plan, which is based on an established model; and(4) according to the standard radiotherapy plan, the master cloud server or the controlled computer conducts conversion to generate a specific radiotherapy plan that matches specific radiotherapy equipment,wherein generating the specific radiotherapy plan comprises introducing the standard radiotherapy plan, and using a dose-volume histogram and isodose line in the standard radiotherapy plan as constraint conditions for optimization of the specific radiotherapy plan, and recomputing a final specific radiotherapy plan based on field parameters of the standard radiotherapy plan,wherein the recomputing comprises one or a combination of dose computation or reverse optimization, wherein the reverse optimization comprises use of one or a combination of a direct subfield optimization method and a flux map optimization method.

According to the present invention, preferably, the patient image includes one or a combination of a CT image, a MRI image, and a PET image.

The medical order data include one or a combination of target radiotherapy dose, DVH curve, and radiotherapy dose constraint value of each organ.

The delineation is automatic delineation, semi-automatic delineation, or manual delineation.

After the step (4), a step (5) of quality assurance (QA) is further included, before treatment of the patient, whether the converted specific radiotherapy plan is correct is verified by the QA; if correct, executing the specific radiotherapy plan; if not, returning to the step (4) to regenerate a new specific radiotherapy plan according to the standard radiotherapy plan.

Between the step (3) and step (4), the method further includes a step of selecting a radiotherapy equipment for conversion according to the congestion situation of the radiotherapy equipment: preferentially converting the standard radiotherapy plan to a radiotherapy equipment currently idle or to a radiotherapy equipment with few task to be performed or to a radiotherapy equipment user-defined for conversion.

When a standard radiotherapy plan is generated or when a radiotherapy plan matching a specific radiotherapy equipment is generated through conversion, a process queue conversion or a user-defined priority of a computation task is performed according to available computing resources.

Before the step where a radiotherapy plan matching a specific radiotherapy equipment is generated through conversion according to the standard radiotherapy plan, the method further includes the following step: comparing parameter coincidence between the standard radiotherapy equipment and the specific radiotherapy equipment to be matched, wherein if the parameter coincidence meets requirement of a predetermined threshold, the standard radiotherapy plan is directly used as the final radiotherapy plan, and if not, proceed to the step (4).

Parameters to be compared between the standard radiotherapy equipment and the specific radiotherapy equipment to be matched include a source parameter and a multi-leaf collimator parameter.

The source parameter is obtained by comparing dose measurement characteristic data of the source in a uniform or non-uniform medium; the dose measurement characteristic data is obtained by a three-dimensional dose curve.

The multi-leaf collimator parameter includes leaf size and pair number, maximum open field size, and whether to allow interleaving.

In the step (4), when the standard radiotherapy plan is converted into the specific radiotherapy plan, the conversion is set to generate one or more specific radiotherapy plans that respectively match one or more specific radiotherapy equipment.

The present invention also provides a computer-readable storage medium storing one or more programs, the one or more programs including instructions, the instructions being adapted to be loaded from a memory and execute the above-mentioned standardized cloud radiotherapy planning method.

The invention has the following beneficial effects:

the present invention provides a standardized cloud radiotherapy planning method, which converts a standard radiotherapy plan into a specific radiotherapy plan through information such as patient data and a patient image, thereby avoiding delay of treatment of patients and idle resources for treatment in the case where a certain type of machine in the hospital fails while other machines are available. Through automatic delineation and formulation of automatic radiotherapy plan (TPS), it can also balance the difference in the treatment level of doctors in different hospitals or regions, and also reduce the workload of oncologists and physicists. The computation task of the radiotherapy plan is assigned to the controlled computer through the master cloud server, which makes it possible to apply the "gold standard" for clinical radiotherapy dose computation, i.e., the dose computation simulated based on Monte Carlo particle transport, to clinical use. In addition, the dose-volume histogram (DVH) and/or isodose line in the standard radiotherapy plan are close to the real situation. Therefore, taking the DVH curve and/or isodose line in the standard radiotherapy plan as input values for dose optimization in subsequent specific radiotherapy plans can greatly reduce the generation time of subsequent specific radiotherapy plans.

BRIEF DESCRIPTION OF THE DRAWINGS

FIG. 1 is a schematic structural diagram of a standardized cloud radiotherapy planning system in a preferred embodiment of the present invention.

FIG. 2 is a flowchart of a standardized cloud radiotherapy planning method in a preferred embodiment of the present invention.

FIG. 3 is a flowchart of a standardized cloud radiotherapy planning method in another preferred embodiment of the present invention.

FIG. 4 is a flowchart of a standardized cloud radiotherapy planning method in yet another preferred embodiment of the present invention.

FIG. 5 is a schematic diagram of a Monte Carlo-based grid parallel dose computation principle in an exemplary embodiment of the present invention.

DETAILED DESCRIPTION OF THE EMBODIMENT(S) OF THE INVENTION

A standardized cloud radiotherapy planning method and system are herein described, by which method a standard radiotherapy plan is converted into a specific radiotherapy plan in a cloud radiotherapy planning system through information such as patient data and a patient image.

The present invention is further described below with reference to the drawings and embodiments. Obviously, the described embodiments are only a part of embodiments of the present invention, but not all the embodiments. Based on the embodiments of the present invention, all other embodiments obtained by a person ordinarily skilled in the art without creative efforts shall fall within the protection scope of the present invention.

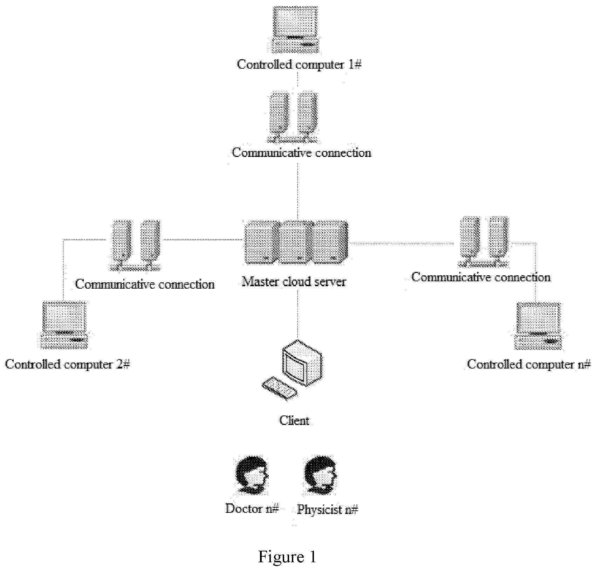

FIG. 1 is a schematic structural diagram of a cloud radiotherapy planning system according to an embodiment of the present invention. The cloud radiotherapy planning system includes a master cloud server, a network communication module, a client, and a controlled computer, wherein the master cloud server, the controlled computer, and the client are communicatively connected through the network communication module; preferably, a data communication channel is established between the master cloud server, the controlled computer and the client through the DICOM (Digital Imaging and Communications in Medicine) protocol. The master cloud server is used to define the computation phantom, delineate a target area and define computation parameters, decompose the computation task into subtasks, optimize the assignment and schedule of tasks, and monitor the execution of the controlled computer; the controlled computer is used to receive the operation instructions sent by the master cloud server, determine task execution, perform computation tasks, and feed computation progress and computation results back, wherein the computation results include specific radiotherapy plans, computation progress, etc.; the client is used to upload patient images, patient data or clinical doses to the master cloud server and check the results of the radiotherapy plan.

The master cloud server defines computation parameters, optimizes assignment and schedule of tasks, and monitors execution of the controlled computer, wherein said optimizing assignment and schedule of tasks is determined by establishing an optimization model, which includes optimization goals and constraint conditions; optionally, the optimization goals include minimum completion time, maximum number of tasks completed, minimum cost, or one or more combinations thereof according to such weights as task priority level, urgency level, etc. Constraint conditions include determining the number of current tasks, distribution of available network, distribution of available controlled computers or distribution of task completion rates of the controlled computers; wherein said optimizing assignment and schedule of tasks includes the following steps:

a) optimization model parameter initialization: including initialization of parameters related to defining the optimization goals and determining the constraint conditions;
b) iterative solution using optimization algorithm: iterative solution using optimization algorithm; wherein optional optimization algorithms include gradient algorithm, conjugate gradient method, Newton method, quasi-Newton method, multiscale algorithm, interior point method, simple method, genetic algorithm, ant colony algorithm, and particle swarm algorithm; and
c) output of results: the optimization results include the priority of the assigned tasks, the network resources used, and the computer resources.

The master cloud server defines a computation phantom, delineates a target area, and defines computation parameters; the master cloud server decomposes, allocates, and schedules computation tasks according to the current user's computation requirements, wherein the assignment and schedule of the computation tasks include one or more of sending a computation task to the controlled computer, closing a computation task, transferring a computation task, switching on and off management, task priority management, or task security management.

The master cloud server monitors the controlled computers, and when any one of the controlled computers is found to be out of contact, the task of the controlled computer is reassigned to another controlled computer. The monitoring method includes actively sending or passively receiving heartbeat packets, actively requesting or passively receiving computation progress, actively requesting or passively receiving computation result related information.

The cloud radiotherapy planning system of this embodiment includes several controlled computers. After receipt of the task assigned by the master cloud server, the controlled computer firstly determine whether the task is performable. If it is determined that the assigned task cannot be completed, the controlled computer feeds the current task status back to the master cloud server and requests the master cloud server to schedule the assigned task to another controlled computer; if it is determined that the assigned task is performable, the controlled computer continues the steps of performing computation tasks (including subtasks, etc.), and feeding the computation progress and computation results back. The computation tasks performed by the controlled computer for formulating a radiotherapy plan include dose computation and/or dose optimization; the controlled computer performing the computation tasks includes decomposition of the tasks into subtasks and execution of the subtasks; optionally, the controlled computer decomposes the tasks into one or more of the following subtasks: GPU parallel tasks, CPU parallel tasks, or CPU-GPU hybrid parallel tasks.

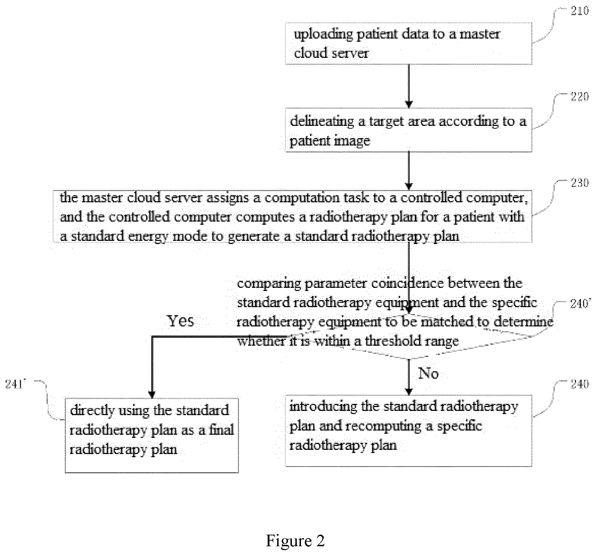

The cloud radiotherapy planning system provided by the present invention can be accessed by multiple users at the same time. As shown in FIG. 1, users (such as doctors, physicists or technicians, etc.) access the master cloud server through the client to define the computing phantom, delineate the target area and define the computation parameters, and check through the client the conversion progress and the specific radiotherapy plan results obtained by the conversion. In this embodiment, it is further preferred that the user may also select and set the model of the specific radiotherapy equipment to be converted and the like through the client. FIG. 2 is a flowchart of a standardized cloud radiotherapy planning method in an exemplary embodiment of the present invention. A standardized cloud radiotherapy planning method suitable for execution in a standardized cloud radiotherapy planning system includes the following steps:

Step 210: patient data is uploaded to the master cloud server, where the patient data include a patient image and medical order data; the patient image include one or a combination of a CT image, a MRI image, or a PET image; the medical order data include one or a combination of target radiotherapy dose, DVH curve and radiation dose constraint value of each organ;

Step 220: the target area is delineated according to the patient image;

Optionally, the method of target area delineation is automatic delineation, semi-automatic delineation, or manual delineation, wherein the automatic delineation of target areas pertains to the prior art. For example, refer to the patent with a publication number CN103247046B and a title of "A method and device for automatically delineating target areas in a radiotherapy plan", in which, with a physicist's manual delineation of a certain target area as prior knowledge, automatic spread of the contour is achieved using circular two-dimensional tomographic registration; or refer to "Automatic delineation evaluation of nasopharyngeal carcinoma target area" (" Sichuan Medical" June 2015 Vol. 36 (No. 6), p762-p765); or refer to Ronneberger, O., Fischer, P., & Brox, T. (2015, October). U-net: Convolutional networks for biomedical image segmentation. In International Conference on Medical Image Computing and Computer-Assisted Intervention (pp.234-241).

Step 230: the master cloud server decomposes the computation task and assigns it to the controlled computer, and the controlled computer computes the patient's radiotherapy plan using a standard radiotherapy equipment mode to generate a standard radiotherapy plan;

In this embodiment, the standard radiotherapy equipment is user-defined radiotherapy equipment or an established model selected as the standard radiotherapy equipment. It is further preferred that the radiotherapy equipment of the model with the largest number is used as the standard radiotherapy equipment, thereby reducing probability of a standard radiotherapy plan being converted into a specific radiotherapy plan and therefore further reducing the amount of computation. The radiotherapy plan obtained based on computation of the standard radiotherapy equipment in the present invention is a standard radiotherapy plan.

The equipment parameters that need to be defined for the above-mentioned user-defined radiotherapy equipment include a source parameter, a multi-leaf collimator parameter, a tungsten gate parameter, and the like. The source parameter includes position of the radioactive source, energy spectrum, direction of movement, type of particles, whether to use a flattening filter, etc.; the said flattening filter is used to reduce intensity in the middle of rays, so as to level the rays. There would be a 3F mode if the flattening filter is not used, where the intensity around the middle of the beam is large, presenting Gaussian distribution; if the flattening filter is used, there would be a 2F mode, where the intensity around the center of the beam is flat and uniform; the multi-leaf collimator parameter includes blade size and pair number, maximum open field size, and whether to allow staggering, etc.; the tungsten gate parameter includes maximum open field size of the tungsten gate.

The computation task in this step is to generate a standard radiotherapy plan. Process of generating the standard radiotherapy plan includes dose computation and/or dose optimization.

Optionally, dose computation parameters of the standard radiotherapy plan include a geometric phantom (determined by a target area image, where the image can be selected from one or a combination of a CT image, an MRI image, a PET image, etc.), medical order data, radiography field size, irradiation direction, source parameter, total number of tracking particles, electron cut-off energy, photon cut-off energy, bremsstrahlung segmentation, range exclusion, and electron segmentation, where the source parameter includes location of the radioactive source, energy spectrum, direction of movement, type of particles, whether to use a flattening filter, etc.

Optionally, existing radiation dose computation models include: a Monte Carlo computation model, an Acuros XB dose computation model (used by a Varian system), and a convolutional superposition dose computation model. Optionally, the convolutional superposition dose computation model further includes Collapse Cone Convolution algorithms (CCC, such computation models is applied in, for example, Pinnacle, CMS, XiO, etc.), and Analytical Anisotropic Algorithm (AAA), and Pencil Beam Model (PBM).

Preferably, in this embodiment, generating a standard radiotherapy plan is a computation task, and there are following optional method for decomposing a computation task into subtasks:

Method I: Splitting a computation task into several subtasks by using a flux map. Specifically, an arbitrary cross section of a beam in an incident direction is divided into a two-dimensional flux grid, and a region of interest in a patient image is divided into a three-dimensional voxel grid. Then, the $i^{th}$ two-dimensional flux grid contributes to the $j^{th}$ voxel with a dose $D_{ij}$; computation task of each dose $D_{ij}$ is a subtask. Optionally, each subtask is assigned as a GPU parallel task, a CPU parallel task, or a CPU-GPU hybrid parallel task.

Or method II: Setting a dose computation or a dose optimization of a beam as a subtask in an intensity modulated radiation therapy (IMRT) mode or a 3D conformal radiation therapy (3D-CRT) mode. In this embodiment, the mode in which the master cloud server allocates computation tasks to the controlled computer includes a single plan mode and a multi-plan mode. The single plan mode is an execution mode of a single radiotherapy plan in the cloud radiotherapy planning system; the multi-plan mode is a number of (equal to or more than 2) radiotherapy plans to be executed at the same time, which radiotherapy plans can be from different patients or can be multiple radiotherapy plans for the same patient.

Mode 1: Single plan mode

The method of allocating the radiotherapy plan computation task in the single plan mode can be implemented by the following steps:

> (1) the master cloud server computes currently available computing resources;
> (2) the master cloud server decomposes the computation task into subtasks; and the computing resources required for completion of the subtasks are computed; and
> (3) the master cloud server assigns the subtasks to the controlled computer. The computation mode of the subtasks is determined according to a goal preset by the user; optionally, the computation mode includes a GPU parallel task, a CPU parallel task, or a CPU-GPU hybrid parallel task; the goal preset by the user may include computation time of the subtasks and/or service cost of computing resources required by the subtasks.

Mode 2: Multi-plan Mode

The method of allocating the radiotherapy plan computation task in the multi-plan mode can be determined by the following steps:

> (1) computing currently available computing resources;
> (2) determining priority level of each radiotherapy plan computation task to be executed; optionally, evaluation index of the priority level includes criticality of patient's illness, time sequence of joining the queue and others;
> (3) determining execution order of multiple radiotherapy plan computation tasks according to the priority level of each radiotherapy plan computation task and the computing resources; and

> (4) decomposing into subtasks by the master cloud server according to execution order of the radiotherapy plan computation tasks and assigning the subtasks to the controlled computer; after a previous computation task is assigned, the next radiotherapy plan computation task is decomposed into several subtasks and assigned to the controlled computer, preferably, the subsequent radiotherapy plan computation subtasks are assigned to the computer with fewer or less to-be-executed computation tasks or the idle controlled computer; optionally, the computation mode of each subtask includes GPU parallel task, CPU parallel task, or CPU-GPU hybrid parallel task; it is available to minimize completion time or the cost of the computation task according to the user's preset goals.

Optionally, the standard radiotherapy plan in this embodiment may be generated in the following two methods:

Method I: Obtaining a standard radiotherapy plan by flux map optimization (FMO):

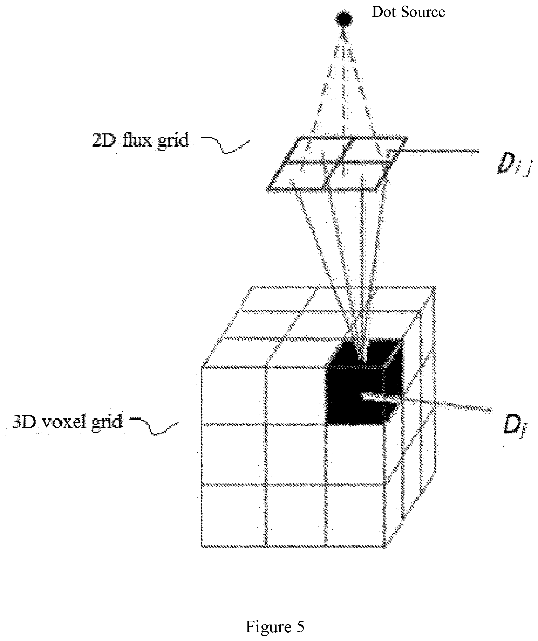

Fig. 5 is a schematic diagram of dose computation based on Monte Carlo-based grid parallel dose computation principle in an exemplary embodiment of the present invention. 3D images of patients or phantoms are divided into 3D grids based on patient images, where each 3D grid is a voxel, and a region of interest is selected from the 3D grids; preferably, a Monte Carlo computation region is determined based on the region of interest, that is, setting a grid within a valid electronic range around the region of an interest and a grid where the region of interest is located as the computation region or directly taking the region of interest as the computation region; any cross section in an incident direction is divided into 2D flux grids, where $D_{ij}$ is a dose contributed by the $i^{th}$ flux grid to the $j^{th}$ voxel; the weight corresponding to each 2D flux grid is $\omega_i$; Monte Carlo dose computation parameters and/or phantom parameters are input; radiation dose of particles in each voxel is computed based on the Monte Carlo particle transport principle and the computation results are normalized; and then the normalized computation results of all grid doses in the computation region are superposed to obtain a total radiation dose. The dose of a single voxel grid $D_j$ is:

$$D_j = \sum_{i=1}^{n} D_{ij} \omega_i (j = 1, 2, ..., m) \qquad (1)$$

wherein

> i is a mark number of 2D flux grids,
> n is the total number of the flux grids,
> j is a mark number of 3D voxels,
> m is the total number of voxels,
> $\omega_i$ is the weight of each flux grid in the Monte Carlo algorithm,
> $D_{ij}$ is the dose contributed by the $i^{th}$ flux grid to the

$j^{th}$ voxel,

$D_j$ is the total dose deposited on the $j^{th}$ voxel.

According to the $D_{ij}$ obtained by the computation, the weight of each of the above 2D flux grids are optimized by an optimization target and further through the Flux Map Optimization; a final inverse dose optimization result is obtained.

Dose distribution in each voxel in the region of interest is obtained through computation, thereby determining an isodose line and a dose-volume histogram (DVH); the dose-volume histogram presents in a graph the relation between dose to which the target area lesion and other key organs are subject and volume, indicating how much dose is at least radiated to an organ of a certain volume. With the dose-volume histogram, dose of each voxel can be obtained through statistics, and then the voxels with the same dose are cumulated to obtain a volume value of the corresponding dose, thereby obtaining a dose-volume histogram of the lesion or organ.

According to the obtained finally inversely optimized 2D flux grid weights, a leaf sequence of each subfield of the field opening shape including MLC and tungsten gate positions is determined to obtain a standard radiotherapy plan.

Method II: Automatically generate a standard radiotherapy plan according to a set model by a machine learning method; for the generation of the standard radiotherapy plan by a machine learning method, please refer to Dose Prediction with U -net: A Feasibility Study for Predicting Dose Distributions from Contours using Deep Learning on Prostate IMRT Patients [J] .2017, Dan N, Long T, Jia X, et a. According to the dose distribution information contained in the radiotherapy plan obtained through machine learning, dose volume histograms and isodose lines can be obtained for subsequent optimization to generate constraint conditions for specific radiotherapy plan steps.

Step 240: The master cloud server or the controlled computer conducts conversion according to the standard radiotherapy plant to generate a radiotherapy plan that matches the specific radiotherapy equipment. The "matching" in the present invention is that the generated specific radiotherapy plan can be executed in the corresponding specific radiotherapy equipment. This step particularly includes:

introducing a standard radiotherapy plan, and using dose-volume histograms, isodose lines, and hardware parameters of equipment with a certain model as constraint conditions to recompute a final specific radiotherapy plan based on field parameters of the standard radiotherapy plan.

Optionally, when a standard radiotherapy plan is converted into a specific radiotherapy plan, the conversion is set to generate one or more specific radiotherapy plan that respectively match one or more specific radiotherapy equipment. That is, when a standard radiotherapy plan is converted into a specific radiotherapy plan, a setting could be made so that the standard radiotherapy plan is converted into multiple specific radiotherapy plans (also known as redundant conversion) that match the specific radiotherapy equipment respectively or converted into a specific radiotherapy plan (also known as specific conversion). For redundant conversion, upon using conditions of the predetermined multiple specific radiotherapy equipment, users can make selection and deploy such that patients are treated with any one of the above specific radiotherapy equipment. Redundant conversion is suitable for situations with abundant computer resources but no so high real-time requirements. In the case of high real-time requirements, a specific conversion mode can be used to complete the conversion from a standard radiotherapy plan to a specific radiotherapy plan as soon as possible.

The specific radiotherapy plan includes a dose volume histogram (DVH), an isodose line, an execution sequence, an opening shape of each subfield, and an execution time of each subfield, which match the specific radiotherapy equipment. The above-mentioned hardware parameter constraint conditions include presence or absence of a tungsten gate, maximum opening size of the tungsten gate, and moving direction of a multi-leaf collimator, blade thickness, maximum opening position, number of blade pairs, leakage and transmission, etc.

The recomputing includes one or a combination of dose computation and/or inverse optimization; wherein, optionally, the inverse optimization includes using one or combination of a direct aperture optimization (DAO) method and a flux map optimization (FMO) method, etc. The DVH curve and/or isodose line of the standard radiotherapy plan are/is used as constraint conditions for the optimization of the specific radiotherapy plan. Because the DVH curve and the isodose line of the standard radiotherapy plan are rather similar to or the same as the final DVH curve and isodose line of the specific radiotherapy plan, the speed of dose optimization in the specific radiotherapy plan can be accelerated.

Optionally, the standard radiotherapy plan in this embodiment may be converted into a dynamic multi-leaf collimator (DMLC) radiotherapy plan, a static multi-leaf collimator (SMLC) radiotherapy plan, a volumetric-modulated arc therapy (VMAT) plan or a constant dose rate intensity modulated art therapy (IMAT) plan according to the model of the specific radiotherapy equipment to be converted.

1. Generating a dynamic multi-leaf collimator (DMLC) radiotherapy plan for specific radiotherapy equipment through a standard radiotherapy plan, including the following steps:
Taking DVH and/or isodose lines of a standard radiotherapy plan as constraint conditions, combined with mechanical constraint conditions of specific radiotherapy equipment (including thickness of the multi-leaf collimator, leaf distribution of the multi-leaf collimator, and the maximum opening position); generating MLC motion sequence through MLC sequence optimization algorithm, where an optimization method is to decompose the flux map, set the number of decompositions, the minimum execution time, the thickness of the multi-leaf collimator blades, the distribution, the maximum opening and other related constraint conditions to generate execution sequence using an optimization algorithm such as the gradient method.

2. Generating a static multi-leaf collimator (SMLC) radiotherapy plan for specific radiotherapy equipment through a standard radiotherapy plan, including the following steps:

Taking DVH and/or isodose lines of a standard radiotherapy plan as constraint conditions, and shape of the subfield opening of the standard radiotherapy plan as an initial condition; directly generating an opening shape of MLC using an optimization algorithm according to thickness, distribution, and maximum opening of a multi-leaf collimator of specific radiotherapy equipment. Optionally, the above optimization algorithm is a direct aperture optimization (DAO).

3. Generating a volumetric-modulated arc therapy (VMAT) plan for specific radiotherapy equipment through a standard radiotherapy plan, including the following steps:

Taking DVH and/or isodose lines of a standard radiotherapy plan as constraint conditions, combined with mechanical constraint conditions of specific radiotherapy equipment (including thickness of the multi-leaf collimator, leaf distribution of the multi-leaf collimator, and the maximum opening position); dividing irradiation of specific radiotherapy equipment in equal angles; optimizing subfield opening shape and dwell time of the multi-leaf collimator at each angle with the optimization algorithm. Optionally, the above optimization algorithm is DAO.

4. Generating a constant dose rate intensity modulated art therapy (IMAT) plan for specific radiotherapy equipment through a standard radiotherapy plan, wherein the dose rate is the radiation dose per unit time, including the following steps:

Taking DVH and/or isodose lines of a standard radiotherapy plan as constraint conditions, combined with mechanical constraint conditions of specific radiotherapy equipment (including thickness of the multi-leaf collimator, leaf distribution of the multi-leaf collimator, and the maximum opening position); dividing irradiation in equal angles; optimizing subfield opening shape and dwell time of the multi-leaf collimator at each angle with the optimization algorithm. Optionally, the above optimization algorithm is DAO.

Optionally, before the step 240, a step 240' is further included to compare whether parameter coincidence between standard radiotherapy equipment and equipment to be matched is within a threshold range. If the parameter coincidence meets a preset threshold requirement, the standard radiotherapy plan is directly used as the final specific radiotherapy plan in the step 241', otherwise turned to step 240;

The equipment parameters to be compared include a source parameter, a multi-leaf collimator parameter, $D_{ij}$ value under the same conditions, whether the grating model matches, the maximum opening position of the tungsten gate, and the like. Further, the source parameter includes source energy spectrum, position, direction, particle type, whether to use a flattening filter, etc.; the multi-leaf collimator parameter includes leaf size and pair number, maximum open field size, whether to allow staggering, etc. When the above parameters are the same or the difference thereof is within a preset threshold of coincidence, it is determined that the parameter coincidence between the standard radiotherapy equipment and the specific radiotherapy equipment to be matched is within a threshold range. Optionally, the energy spectrum of the standard radiotherapy equipment and that of the specific radiotherapy equipment are compared using a similarity method. When the similarity between the above energy spectrums is within a preset threshold range, they are considered similar.

Optionally, in this embodiment, the coincidence of source parameters is obtained by comparing the characteristics data (three-dimensional dose curve) of dose measurement of the source in a uniform or non-uniform medium, specifically including the following steps:

(1) comparing similarity between the characteristics data of dose measurement of the specific radiotherapy equipment and that of dose measurement of the standard plan one by one;
(2) computing comprehensive similarity, weighting all the similarities through preset weights to obtain a comprehensive similarity; and
(3) affirming consistency if the comprehensive similarity satisfies a preset threshold.

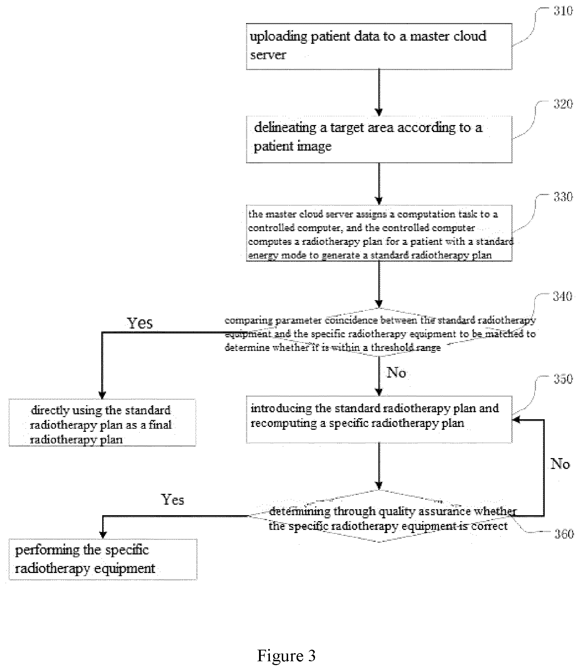

In the embodiment shown in FIG. 3, based on the embodiment shown in FIG. 2, a quality assurance (QA) step is further included after step (4), to enable a patient to verify by QA before treatment whether the converted plan is right or not.

FIG. 3 is a flowchart of a standardized cloud radiotherapy planning method in another preferred embodiment of the present invention, which is suitable for execution in a standardized cloud radiotherapy planning system, including the following steps:

Step 310: patient data is uploaded to a master cloud server, where the patient data include a patient image and medical order data; the patient image includes one or a combination of a CT image, an MRI image, or a PET image; the medical order data include one or a combination of target radiotherapy dose, DVH curve, and radiotherapy dose constraint value of each organ;

Step 320: a target area is delineated according to the patient image; the delineation is an automatic delineation or a manual delineation;

Step 330: the master cloud server assigns a computation task to a controlled computer, and the controlled computer computes the patient's radiotherapy plan using a standard radiotherapy equipment mode to generate a standard radiotherapy plan;

Optionally, step 340 may be included, comparing compare whether parameter coincidence between standard radiotherapy equipment and equipment to be matched is within a threshold range; the parameter includes a source parameter and a multi-leaf collimator parameter; wherein the source parameter is obtained by comparing characteristic data of dose measurement of the source in a non-uniform medium and that in a uniform medium (three-dimensional dose curve); the multi-leaf collimator parameter includes leaf size and pair number, maximum open field size, and whether to allow staggering; in step 340, if the parameter coincidence meets a preset threshold, the standard radiotherapy plan is directly used as the final radiotherapy plan, otherwise turned to step 350;

Step 350: the master cloud server or the controlled computer conducts conversion to generate a radiotherapy plan that matches the specific radiotherapy equipment according to the standard radiotherapy plan. Preferably, this step further includes:

introducing the standard radiotherapy plan, and using a dose-volume histogram and isodose line in the standard radiotherapy plan as constraint conditions to recompute a final radiotherapy plan based upon field parameters of the standard radiotherapy plan; the recomputing includes one or a combination of dose computation or inverse optimizations; wherein, the inverse optimization includes one or a combination of a direct subfield optimization method or a flux map optimization method; and

Step 360 is a quality assurance (QA) process, that is, verifying by the quality assurance before executing the specific radiotherapy plan for a patient whether the converted specific radiotherapy plan is correct. Optionally, during the quality assurance step, execution target of the specific radiotherapy plan is replaced with a phantom made of solid water or other human tissue replacement materials, to test whether radiation dose and dose distribution received in the phantom meet requirements of a medical order. When the requirements of the medical order are met, the specific radiotherapy plan is executed, and if not, return to step 350 to regenerate a new specific radiotherapy plan.

The standardized cloud radiotherapy planning method shown in FIG. 3 is the same as the method shown in FIG. 2 except for the contents described above.

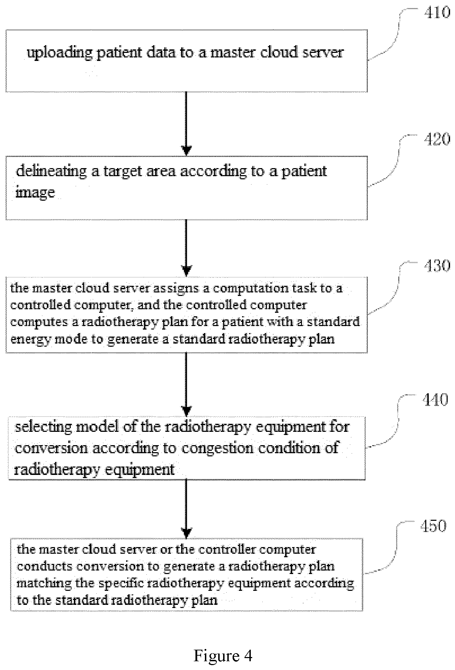

In the embodiment shown in FIG. 4, a flowchart of a standardized cloud radiotherapy planning method, in the process of formulating a radiotherapy plan, further includes a step of selecting the model of radiotherapy equipment for conversion according to use status of the radiotherapy equipment, priorly selecting the equipment idle or having few tasks or making selection as defined by users. The standardized cloud radiotherapy planning method of this embodiment specifically includes the following steps:

Step 410: patient data is uploaded to a master cloud server, where the patient data include a patient image and medical order data; the patient image includes one or a combination of a CT image, an MRI image, or a PET image; the medical order data include one or a combination of target radiotherapy dose, DVH curve and radiation dose constraint value of each organ;

Step 420: a target area is delineated according to the patient image; the delineation is an automatic delineation or a manual delineation;

Step 430: the master cloud server assigns a computation task to a controlled computer, and the controlled computer computes the patient's radiotherapy plan using a standard radiotherapy equipment mode to generate a standard radiotherapy plan;

Step 440: model of radiotherapy equipment to be converted is selected according to the congestion situation of the radiotherapy equipment, and preferentially the standard radiotherapy plan is converted to a radiotherapy equipment currently idle or to a radiotherapy equipment with few task to be performed or to a radiotherapy equipment with a model user-defined for conversion;

Step 450: the master cloud server or the controlled computer conducts conversion to generate a radiotherapy plan that matches the specific radiotherapy equipment according to the standard radiotherapy plan. This step further includes introducing the standard radiotherapy plan, and using dose-volume histogram and isodose lines in the standard radiotherapy plan as a constraint condition, to recompute a final radiotherapy plan based on the field parameters of the standard radiotherapy plan; recomputing one or a combination of dose computation or reverse optimization; wherein the reverse optimization includes use of one or a combination of a direct subfield optimization method and a flux map optimization method; and

Optionally, a step 460 of quality assurance (QA) (not shown in FIG. 4) is further included, verifying through QA before treatment whether the converted plan is correct. Optionally, when performing the quality assurance step, execution target of the specific radiotherapy plan is replaced with a phantom made of solid water or other human tissue replacement materials, to test whether radiation dose and dose distribution received in the phantom meet requirements of a medical order. When the requirements of the medical order are met, the specific radiotherapy plan is executed, and if not, return to step 350 to regenerate a new specific radiotherapy plan.

The standardized cloud radiotherapy planning method shown in FIG. 4 is the same as the method shown in FIG. 2 or FIG. 3 except for the content described above.

The present invention also provides a computer-readable storage medium storing one or more programs. The one or more programs include instructions, which are adapted to be loaded from the memory and execute the above-mentioned standardized cloud radiotherapy planning method. The method includes steps:

patient data is uploaded to a master cloud server, wherein the patient data include a patient image and medical order data;
target area is delineated according to the patient image;
the master cloud server decomposes the computation task and assigns it to a controlled computer, and the controlled computer calculates the patient's radiotherapy plan using a standard radiotherapy equipment mode to generate a standard radiotherapy plan; and
the master cloud server or the controlled computer converts the standard radiotherapy plan to generate a specific radiotherapy plan that matches the specific radiotherapy equipment according to the assignment of the master cloud server.

For the steps implemented when the above computer program is executed by the processor, refer to the aforementioned description of the embodiments of the method and the system. In addition, upon the premise without conflict, the contents of the embodiments of the system and those of the embodiments of the methods can complement each other and will not be repeated here.

These computer program instructions may also be stored in a computer-readable memory capable of directing a computer or other programmable data processing device to work in a particular manner such that the instructions stored in the computer-readable memory produce a manufactured article including an instruction device The instruction device implements the functions specified in one or more processes of a flowchart and/or one or more blocks of a block diagram.

These computer program instructions may also be loaded on a computer or other programmable data processing device, so that a series of operation steps are performed on the computer or other programmable device to generate a computer-implemented process, and thus the instructions executed on the computer or other programmable device provide steps for implementing the functions specified in one or more processes of a flowchart and/or one or more blocks of a block diagram.

Computer-readable media include permanent and non-permanent, removable and non-removable media. Information storage can be accomplished by any method or technology. Information may be computer-readable instructions, data structures, modules of a program, or other data. Examples of computer storage media include, without limitation, phase change memory (PRAM), static random access memory (SRAM), dynamic random access memory (DRAM), other types of random access memory (RAM), read-only memory (ROM), electrically erasable programmable read-only memory (EEPROM), flash memory or other memory technologies, read-only disc read-only memory (CD-ROM), digital versatile disc (DVD) or other optical storage, magnetic tape cartridges, magnetic tape magnetic disk storage or other magnetic storage devices or any other non-transmission media, which may be used to store information that can be accessed by computing devices. As defined herein, computer-readable media does not include temporary computer-readable media (transitory media), such as modulated data signals and carrier waves.

Through the standardized cloud radiotherapy planning method provided by the foregoing embodiments of the present invention, it is possible to avoid a delay in treatment time for a patient and idleness of treatment resources when a certain type of machine in a hospital fails and other machines are idling; a master cloud service assigning a computation task of a radiotherapy plan to a controlled computer makes it possible to apply the "gold standard" of radiotherapy dose computation, i.e., Monte Carlo particle transport simulation dose computation, which is difficult for clinical use, greatly saving the time of formulating a radiotherapy plan and waiting time of patients, and improving accuracy of dose computation of the radiotherapy plan. In addition, through automatic delineation and automatic TPS formulation, it is also possible to balance the differences in treatment levels of doctors in different hospitals or regions, and also reduce the workload of oncologists and physicists.

The foregoing description of the embodiments is to facilitate understanding and application of the present invention by those skilled in the art. It will be apparent to those skilled in the art that various modifications can be easily made to these embodiments and the general principles described herein can be applied to other embodiments without creative effort. Therefore, the present invention is not limited to the embodiments herein, and improvements and modifications made by those skilled in the art that do not depart from the scope of the present invention shall fall into the scope of the present invention.

## Claims

1. A standardized cloud radiotherapy planning method suitable for execution in a standardized cloud radiotherapy planning system, comprising the following steps:

   (1) patient data is uploaded to a master cloud server, wherein the patient data comprises a patient image and medical order data;
   (2) a target area is delineated on the basis of the patient image;
   (3) the master cloud server decomposes a computation task and assigns it to a controlled computer, and the controlled computer uses a standard radiotherapy equipment mode to compute a radiotherapy plan for a patient to generate a standard radiotherapy plan, which is based on an established model; and
   (4) according to the standard radiotherapy plan, the master cloud server or the controlled computer conducts conversion to generate a specific radiotherapy plan that matches specific radiotherapy equipment,

   the method being **characterised by** the following steps
   generating the specific radiotherapy plan comprises introducing the standard radiotherapy plan, and using a dose-volume histogram and isodose line in the standard radiotherapy plan as constraint conditions for optimization of the specific radiotherapy plan, and recomputing a final specific radiotherapy plan based on field parameters of the standard radiotherapy plan,
   wherein the recomputing comprises one or a combination of dose computation or reverse optimization, wherein the reverse optimization comprises use of one or a combination of a direct subfield optimization method and a flux map optimization method.

2. The standardized cloud radiotherapy planning method according to Claim 1, wherein the patient image comprises one or a combination of a CT image, a MRI image, or a PET image.

3. The standardized cloud radiotherapy planning method according to Claim 1, wherein the medical order data comprise one or a combination of target radiotherapy dose, DVH curve, and radiotherapy dose constraint value of each organ.

4. The standardized cloud radiotherapy planning method according to Claim 1, wherein the delineation is automatic delineation, semi-automatic delineation, or manual delineation.

5. The standardized cloud radiotherapy planning method according to Claim 1, wherein a quality assurance step is also provided after the step (4), verifying through the quality assurance step before treatment of a patient whether the converted plan is correct or not; if correct, executing the specific radiotherapy plan; and if not, returning to the step (4) to regenerate a new specific radiotherapy plan according to the standard radiotherapy plan.

6. The standardized cloud radiotherapy planning method according to Claim 1, wherein between the step (3) and the step (4), the method further comprises a step of selecting a radiotherapy equipment for conversion according to congestion situation of the radiotherapy equipment, where the standard radiotherapy plan is preferentially converted to a radiotherapy equipment currently idle or to a radiotherapy equipment with few task to be performed or to a radiotherapy equipment as user-defined for conversion.

7. The standardized cloud radiotherapy planning method according to Claim 1, wherein, when the standard radiotherapy plan is generated or before the radiotherapy plan matching the specific radiotherapy equipment is generated through conversion, a process queue conversion is performed according to available computing resources or priority of computation tasks is set by users themselves.

8. The standardized cloud radiotherapy planning method according to Claim 1, wherein said "according to the standard radiotherapy plan, the master cloud server or the controlled computer conducts conversion to generate a specific radiotherapy plan that matches specific radiotherapy equipment" further comprises the following step: comparing parameter coincidence between the standard radiotherapy equipment and the specific radiotherapy equipment to be matched, and using the standard radiotherapy plan directly as the final radiotherapy plan if the parameter coincidence meets requirement of a predetermined threshold, and if not, proceeding to the step (4).

9. The standardized cloud radiotherapy planning method according to Claim 8, wherein parameters to be compared between the standard radiotherapy equipment and the specific radiotherapy equipment to be matched comprise a source parameter and a multi-leaf collimator parameter.

10. The standardized cloud radiotherapy planning method according to Claim 9, wherein the source parameter is obtained by comparing dose measurement characteristic data of the source in a uniform or non-uniform medium; the dose measurement characteristic data is obtained by a three-dimensional dose curve; the multi-leaf collimator parameter comprises blade size and pair number, maximum open field size, and whether to allow staggering.

11. The standardized cloud radiotherapy planning method according to Claim 1, wherein, in the step (4), when the standard radiotherapy plan is converted into the specific radiotherapy plan, conversion is set to generate one or more specific radiotherapy plans that respectively match one or more specific radiotherapy equipment

12. A computer-readable storage medium storing one or more programs, the one or more programs comprising instructions, the instructions being adapted to be loaded from a memory and execute the standardized cloud radiotherapy plan method according to any one of Claims 1-11.

**Patentansprüche**

1. Standardisiertes Cloud-Strahlentherapie-Planungsverfahren, das zur Ausführung in einem standardisierten Cloud-Strahlentherapie-Planungssystem geeignet ist, umfassend die folgenden Schritte:

(1) Patientendaten werden auf einen Master-Cloud-Server hochgeladen, wobei die Patientendaten ein Patientenbild und medizinische Auftragsdaten umfassen;
(2) ein Zielbereich wird auf der Grundlage des Patientenbildes abgegrenzt;
(3) der Master-Cloud-Server zerlegt eine Berechnungsaufgabe und weist sie einem gesteuerten Computer zu, und der gesteuerte Computer verwendet einen Standard-Strahlentherapieausrüstungsmodus, um einen Strahlentherapieplan für einen Patienten zu berechnen, um einen Standard-Strahlentherapieplan zu erzeugen, der auf einem etablierten Modell basiert; und
(4) entsprechend dem Standard-Strahlentherapieplan führt der Master-Cloud-Server oder der gesteuerte Computer eine Umwandlung durch, um einen spezifischen Strahlentherapieplan zu erzeugen, der zu einer spezifischen Strahlentherapieausrüstung passt,
wobei das Verfahren durch die folgenden Schritte gekennzeichnet ist:

das Erzeugen des spezifischen Strahlentherapieplans umfasst das Einführen des Standard-Strahlentherapieplans und das Verwenden eines Dosis-Volumen-Histogramms und einer Isodosis-Linie in dem Standard-Strahlentherapieplan als Randbedingungen für die Optimierung des spezifischen Strahlentherapieplans, und Neuberechnung eines endgültigen spezifischen Strahlentherapieplans auf der Grundlage von Feldparametern des Standard-Strahlentherapieplans,
wobei die Neuberechnung eine oder eine Kombination aus Dosisberechnung oder inverser Optimierung umfasst, wobei die inverse Optimierung die Verwendung einer oder einer Kombination aus einem direkten Teilfeldoptimierungsverfahren und einem Flusskartenoptimierungsverfahren umfasst.

2. Standardisiertes Cloud-Strahlentherapie-Planungsverfahren nach Anspruch 1, wobei das Patientenbild eine oder eine Kombination aus einem CT-Bild, einem MRT-Bild oder einem PET-Bild umfasst.

3. Standardisiertes Cloud-Strahlentherapie-Planungsverfahren nach Anspruch 1, wobei die medizinischen Auftragsdaten eine oder eine Kombination aus Ziel-Strahlentherapie-Dosis, DVH-Kurve und Strahlentherapie-Dosis-Beschränkungswert für jedes Organ umfassen.

4. Standardisiertes Cloud-Strahlentherapie-Planungsverfahren nach Anspruch 1, wobei die Abgrenzung eine automatische Abgrenzung, eine halbautomatische Abgrenzung oder eine manuelle Abgrenzung ist.

5. Standardisiertes Cloud-Strahlentherapie-Planungsverfahren nach Anspruch 1, wobei nach dem Schritt (4) auch ein Qualitätssicherungsschritt vorgesehen ist, der vor der Behandlung eines Patienten überprüft, ob der umgewandelte Plan korrekt ist oder nicht; wenn er korrekt ist, den spezifischen Strahlentherapieplan ausführt; und wenn er nicht korrekt ist, zu Schritt (4) zurückkehrt, um einen neuen spezifischen Strahlentherapieplan gemäß dem Standard-Strahlentherapieplan zu generieren.

6. Standardisiertes Cloud-Strahlentherapie-Planungsverfahren nach Anspruch 1, wobei das Verfahren zwischen dem Schritt (3) und dem Schritt (4) ferner einen Schritt der Auswahl eines Strahlentherapiegeräts für die Umwandlung entsprechend der Überlastungssituation des Strahlentherapiegeräts umfasst, wobei der Standard-Strahlentherapieplan vorzugsweise in ein derzeit ungenutztes Strahlentherapiegerät oder in ein Strahlentherapiegerät mit wenigen auszuführenden Aufgaben oder in ein benutzerdefiniertes Strahlentherapiegerät für die Umwandlung umgewandelt wird.

7. Standardisiertes Cloud-Strahlentherapie-Planungsverfahren nach Anspruch 1, wobei bei der Erzeugung des Standard-Strahlentherapieplans oder vor der Erzeugung des für die spezifische Strahlentherapieausrüstung passenden Strahlentherapieplans durch Konvertierung eine Prozesswarteschlangenkonvertierung entsprechend den verfügbaren Rechenressourcen durchgeführt wird oder die Priorität der Berechnungsaufgaben von den Benutzern selbst festgelegt wird.

8. Standardisiertes Cloud-Strahlentherapie-Planungsverfahren nach Anspruch 1, wobei der Schritt "gemäß dem Standard-Strahlentherapieplan führt der Master-Cloud-Server oder der gesteuerte Computer eine Umwandlung durch, um einen spezifischen Strahlentherapieplan zu erzeugen, der zu einer spezifischen Strahlentherapieausrüstung passt" weiterhin den folgenden Schritt umfasst: Vergleichen der Parameterübereinstimmung zwischen der Standard-Strahlentherapieausrüstung und der spezifischen Strahlentherapieausrüstung, die übereinstimmen soll, und direktes Verwenden des Standard-Strahlentherapieplans als endgültigen Strahlentherapieplan, wenn die Parameterübereinstimmung die Anforderung eines vorbestimmten Schwellenwerts erfüllt, und wenn nicht, Fortfahren mit Schritt (4).

9. Standardisiertes Cloud-Strahlentherapie-Planungsverfahren nach Anspruch 8, wobei die Parameter, die zwischen der Standard-Strahlentherapie-Ausrüstung und der spezifischen, abzustimmenden Strahlentherapie-Ausrüstung verglichen werden sollen, einen Quellenparameter und einen Multi-Lamellen-Kollimatorparameter umfassen.

10. Standardisiertes Cloud-Strahlentherapie-Planungsverfahren nach Anspruch 9, wobei der Quellenparameter durch Vergleich von Dosis-Messungs-Charakteristikdaten der Quelle in einem gleichförmigen oder ungleichförmigen Medium erhalten wird; wobei die Dosis-Messungs-Charakteristikdaten durch eine dreidimensionale Dosiskurve erhalten werden; wobei der Parameter des Multi-Lamellen-Kollimators die Blattgröße und Paaranzahl, die maximale offene Feldgröße und die Frage, ob eine Staffelung zugelassen werden soll, umfasst.

11. Standardisiertes Cloud-Strahlentherapie-Planungsverfahren nach Anspruch 1, wobei im Schritt (4), wenn der Standard-Strahlentherapieplan in den spezifischen Strahlentherapieplan umgewandelt wird, die Umwandlung so eingestellt wird, dass ein oder mehrere spezifische Strahlentherapiepläne erzeugt werden, die jeweils zu einem oder mehreren spezifischen Strahlentherapiegeräten passen.

12. Computerlesbares Speichermedium, auf dem ein oder mehrere Programme gespeichert sind, wobei das eine oder die mehreren Programme Befehle umfassen und die Befehle so ausgelegt sind, dass sie aus einem Speicher geladen werden können und das standardisierte Cloud-Strahlentherapie-Planungsverfahren nach einem der Ansprüche 1 bis 11 ausführen.

**Revendications**

1. Procédé de planification de radiothérapie en nuage standardisée adapté à l'exécution dans un système de planification de radiothérapie en nuage standardisée, comprenant les étapes suivantes :

(1) des données de patient sont téléchargées sur un serveur cloud maître, en ce que les données de patient comprennent une image de patient et des données d'ordre médical ;
(2) une zone cible est délimitée sur la base de l'image de patient ;
(3) le serveur cloud maître décompose une tâche de calcul et l'attribue à un ordinateur commandé, et l'ordinateur commandé utilise un mode d'équipement de radiothérapie standard pour calculer un plan de radiothérapie pour un patient en vue de générer un plan de radiothérapie standard qui est basé sur un modèle établi ; et
(4) selon le plan de radiothérapie standard, le serveur cloud maître ou l'ordinateur commandé effectue une conversion pour générer un plan de radiothérapie spécifique qui correspond à un équipement de radiothérapie spécifique,

le procédé étant **caractérisé par** les étapes suivantes
la génération du plan de radiothérapie spécifique comprend l'introduction du plan de radiothérapie standard, et l'utilisation d'un histogramme dose-volume et d'une ligne d'isodose dans le plan de radiothérapie standard comme conditions de contrainte pour l'optimisation du plan de radiothérapie spécifique, et le recalcul d'un plan de radiothérapie spécifique final sur la base de paramètres de champ du plan de radiothérapie standard,
en ce que le recalcul comprend un calcul ou une combinaison de calcul de dose ou d'optimisation inverse, en ce que l'optimisation inverse comprend l'utilisation d'un procédé ou d'une combinaison d'un procédé d'optimisation de sous-champ direct et un procédé d'optimisation de carte de flux.

2. Procédé de planification de radiothérapie en nuage standardisée selon la revendication 1, en ce que l'image de patient comprend une ou une combinaison d'images TDM, images IRM ou images TEP.

**3.** Procédé de planification de radiothérapie en nuage standardisée selon la revendication 1, en ce que les données d'ordre médical comprennent une ou une combinaison de dose de radiothérapie cible, de courbe DVH, et de valeur de contrainte de dose de radiothérapie de chaque organe.

**4.** Procédé de planification de radiothérapie en nuage standardisée selon la revendication 1, en ce que la délimitation est une délimitation automatique, une délimitation semi-automatique, ou une délimitation manuelle.

**5.** Procédé de planification de radiothérapie en nuage standardisée selon la revendication 1, en ce qu'une étape d'assurance qualité est également prévue après l'étape (4), vérifiant par le biais de l'étape d'assurance qualité avant traitement d'un patient si le plan converti est correct ou non ; s'il est correct, exécutant le plan de radiothérapie spécifique ; et s'il n'est pas correct, retournant à l'étape (4) pour régénérer un nouveau plan de radiothérapie spécifique conformément au plan de radiothérapie standard.

**6.** Procédé de planification de radiothérapie en nuage standardisée selon la revendication 1, en ce qu'entre l'étape (3) et l'étape (4), le procédé comprend en outre une étape de sélection d'un équipement de radiothérapie pour la conversion en fonction de la situation d'encombrement de l'équipement de radiothérapie, le plan de radiothérapie standard étant de préférence converti en un équipement de radiothérapie actuellement inactif ou en un équipement de radiothérapie ayant peu de tâches à effectuer ou en un équipement de radiothérapie dont la conversion est définie par l'utilisateur.

**7.** Procédé de planification de radiothérapie en nuage standardisée selon la revendication 1, en ce que, lorsque le plan de radiothérapie standard est généré ou avant que le plan de radiothérapie correspondant à l'équipement de radiothérapie spécifique ne soit généré par conversion, une conversion de file d'attente de processus est effectuée en fonction des ressources informatiques disponibles ou la priorité des tâches de calcul est définie par les utilisateurs eux-mêmes.

**8.** Procédé de planification de radiothérapie en nuage standardisée selon la revendication 1, en ce que ledit « en fonction du plan de radiothérapie standard, le serveur cloud maître ou l'ordinateur commandé effectue une conversion pour générer un plan de radiothérapie spécifique qui correspond à l'équipement de radiothérapie spécifique » comprend en outre l'étape suivante : comparaison de la coïncidence de paramètres entre l'équipement de radiothérapie standard et l'équipement de radiothérapie spécifique à adapter, et utilisation du plan de radiothérapie standard directement comme plan de radiothérapie final si la coïncidence de paramètres répond aux conditions requises d'un seuil prédéterminé, et si ce n'est pas le cas, passage à l'étape (4).

**9.** Procédé de planification de radiothérapie en nuage standardisée selon la revendication 8, en ce que des paramètres à comparer entre l'équipement de radiothérapie standard et l'équipement de radiothérapie spécifique à adapter comprennent un paramètre source et un paramètre de collimateur multilame.

**10.** Procédé de planification de radiothérapie en nuage standardisée selon la revendication 9, en ce que le paramètre source est obtenu par comparaison des données caractéristiques de mesure de dose dans un milieu uniforme ou non uniforme ; les données caractéristiques de mesure de dose sont obtenues par une courbe de dose tridimensionnelle ; le paramètre de collimateur multilame comprend la taille de lame et le nombre de paires, la taille maximale de champ ouvert, et l'autorisation ou non de l'échelonnement.

**11.** Procédé de planification de radiothérapie en nuage standardisée selon la revendication 1, en ce que, à l'étape (4), lorsque le plan de planification standard est converti en plan de radiothérapie spécifique, la conversion est définie pour générer un ou plusieurs plans de radiothérapie spécifiques qui correspondent respectivement à un ou plusieurs équipements de radiothérapie spécifiques.

**12.** Support de stockage lisible par ordinateur stockant un ou plusieurs programmes, le ou les programmes comprenant des instructions, les instructions étant adaptées pour être chargées à partir d'une mémoire et exécution du procédé de planification de radiothérapie en nuage standardisée selon l'une des revendications 1 à 11.

Figure 1

uploading patient data to a master cloud server ⌐ 210

delineating a target area according to a patient image ⌐ 220

the master cloud server assigns a computation task to a controlled computer, and the controlled computer computes a radiotherapy plan for a patient with a standard energy mode to generate a standard radiotherapy plan ⌐ 230

comparing parameter coincidence between the standard radiotherapy equipment and the specific radiotherapy equipment to be matched to determine whether it is within a threshold range ⌐ 240'

Yes

No

241' ⌐

directly using the standard radiotherapy plan as a final radiotherapy plan

introducing the standard radiotherapy plan and recomputing a specific radiotherapy plan ⌐ 240

Figure 2

| | |
|---|---|
| uploading patient data to a master cloud server | 310 |
| delineating a target area according to a patient image | 320 |
| the master cloud server assigns a computation task to a controlled computer, and the controlled computer computes a radiotherapy plan for a patient with a standard energy mode to generate a standard radiotherapy plan | 330 |
| comparing parameter coincidence between the standard radiotherapy equipment and the specific radiotherapy equipment to be matched to determine whether if is within a threshold range | 340 |

Yes

No

| directly using the standard radiotherapy plan as a final radiotherapy plan | introducing the standard radiotherapy plan and recomputing a specific radiotherapy plan | 350 |

No

Yes

determining through quality assurance whether the specific radiotherapy equipment is correct — 360

performing the specific radiotherapy equipment

Figure 3

410

uploading patient data to a master cloud server

420

delineating a target area according to a patient image

430

the master cloud server assigns a computation task to a controlled computer, and the controlled computer computes a radiotherapy plan for a patient with a standard energy mode to generate a standard radiotherapy plan

440

selecting model of the radiotherapy equipment for conversion according to congestion condition of radiotherapy equipment

450

the master cloud server or the controller computer conducts conversion to generate a radiotherapy plan matching the specific radiotherapy equipment according to the standard radiotherapy plan

Figure 4

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017133654 A

- CN 103247046 B

### Non-patent literature cited in the description

- World Cancer Report 2014. World Health Organization (WHO), 03 February 2014
- *Sichuan Medical,* June 2015, vol. 36 (6), 762-765

- **RONNEBERGER, O. ; FISCHER, P. ; BROX, T.** U-net: Convolutional networks for biomedical image segmentation. *International Conference on Medical Image Computing and Computer-Assisted Intervention,* October 2015, 234-241